(19) Europäisches Patentamt
European Patent Office
Office européen des brevets

(11) **EP 3 895 704 A1**

(12) **EUROPEAN PATENT APPLICATION**

(43) Date of publication:
**20.10.2021 Bulletin 2021/42**

(21) Application number: **21169147.2**

(22) Date of filing: **19.04.2021**

(51) Int Cl.:
**A61K 31/198** (2006.01)       **A61K 33/20** (2006.01)
**A61K 33/42** (2006.01)       **A61P 43/00** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.04.2020 IT 202000008203**

(71) Applicant: **DMF Dietetic Metabolic Food S.R.L.**
**20812 Limbiate (IT)**

(72) Inventors:
• VICENTINI, Titi Giorgio
  **20031 Cesano Moderno (IT)**
• GINGRICH, Susan
  **Ayer, MA 04132 (US)**
• NAIR, Manoj
  **Ayer, MA 04132 (US)**
• SPEARS, Mary
  **Ayer, MA 04132 (US)**

(74) Representative: **Coppo, Alessandro et al**
**Notarbartolo & Gervasi S.p.A.**
**Viale Achille Papa, 30**
**20149 Milano (IT)**

(54) **NUTRITIONAL COMPOSITION FOR THE TREATMENT OF METABOLIC DISEASES**

(57) A composition for preventing or treating a metabolic disease, in particular phenylketonuria, comprising sulfur amino acids and minerals is provided, wherein said sulfur amino acids comprise a combination of methionine and cysteine in a methionine/cysteine ratio of 2.2-2.7: 1 and said minerals comprise sodium, potassium, magnesium, calcium, chlorine, phosphorus. The composition of the invention is formulated to provide a negative PRAL value, preferably with -35 < PRAL < -20 values mEq/day on 100 g of equivalent proteins.

EP 3 895 704 A1

**Description**

FIELD OF THE INVENTION

The present invention relates to a nutritional composition suitable for the dietary treatment of hereditary metabolic diseases.

The present invention originates in the field of nutritional and pharmaceutical products. Specifically, the present invention relates to compositions containing a combination of nutritional substances in amounts suitable to keep within physiological parameters the metabolism of subjects suffering from hereditary metabolic diseases, specifically protein metabolism congenital related disorders, among which phenylketonuria.

BACKGROUND OF THE INVENTION

Hereditary metabolic diseases, also referred to as inborn errors of metabolism, fall within the genetic diseases which originates from total or partial deficiency of a specific enzyme or to disruption thereof.

This deficiency is responsible for slowing down or blocking a metabolic pathway of the human organism, determining a build-up in the body of substances which become, directly or indirectly, mainly responsible for the development of the disease.

One of the most studied and widespread hereditary metabolic diseases is phenylketonuria, also known with the acronym PKU.

Phenylketonuria is a rare recessive autosomal-transmitted hereditary metabolic disease caused by a mutation in the gene encoding hepatic enzyme, phenylalanine hydroxylase (PAH), necessary for phenylalanine (Phe) metabolism, a fundamental amino acid present in most protein-containing food.

The partial or total reduction of the PAH enzyme activity in the organism leads to a build-up of phenylalanine at plasmatic and cerebral level (hyperphenyllalanine), with toxicant and harmful effects for the central nervous system. The plasmatic levels of phenylalanine in the blood determine the severity of the disease, which can be mild, moderate or severe (classic PKU).

The disease consequences may be intellectual disability, microcephaly, motor deficiencies, eczematous rash, autistic spectrum disorders, epilepsy, developmental delay, growth delay, aberrant behaviours and psychiatric symptoms. If not treated, this disease may involve a severe and irreversible mental retardation, in addition to significant cognitive disabilities.

In order to overcome or limit damages to the central nervous system resulting from a phenylalanine build-up in the blood, a strict dietary regime with a low natural protein content is adopted. Diet therapy represents to the present day the key point in the treatment of this disease and is based on three substantial aids adapted to ensure protein needs suitable for an optimal physiological growth of the organism.

The first one provides removing or reducing the protein-based intake from natural proteins such as meat, fish, eggs, pasta. This measure aims at reducing the daily dietary intake of phenylalanine.

The second one provides replacing or supplementing the dietary regime with phenylalanine-free amino acid formulations. The proteins supplied should cover the required daily protein need for the growth and for maintaining the physiological conditions of the human organism and specifically should provide between 52 and 80% of the total protein amount recommended in subjects suffering from phenylketonuria. The third one provides supplementing the dietary regime with micronutrients such as vitamins, minerals, DHA.

The object of the diet therapy is to maintain phenylalanine plasmatic concentrations within the ranges defined as "safe" by the last "The complete European guidelines on phenylketonuria: diagnosis and treatment." (Van Wegberg, A.M.J., MacDonald, A., Ahring, K. et al. (2017) Orphanet J Rare Dis, 12, 162). Such values defined between 120 - 360 $\mu$mol/dL until the age of 12 and between 120 - 600 $\mu$mol/dL from the age of 13 on, ensure a height-weight growth and a physiological neuro-cognitive development, preventing potential damages to the organism in the long term.

It is known from scientific literature that nutrition plays a fundamental role in regulating and activating the mail metabolic processes of our organism.

The intake of food starts the digestive process in the gastroenteric system, with nutrients being absorbed and consequent excretion of hydrogen ions. Such process determines the action by kidneys, which, re-absorbing bicarbonates, represent the main buffer system of our organism, thus contributing to maintain a stable pH.

This regulating mechanism has significant consequences also on the bones, as alterations of the acid-base balance may in fact determine the reduction in the bone mineral density and osteoporosis due to osteoclast activation, with consequent release of calcium and phosphorus to buffer hydrogen ions and keep a plasmatic pH within the physiological ranges.

Considering the strict relation between nutrition and changes in the correspondent metabolic processes, variations in the food scheme may be responsible for substantial modifications of the acid-base balance, in particular encouraging, in presence of highly acid load food schemes, the development of metabolic acidosis to the detriment of maintaining normal organic functions (Remer, T. (2001). Influence of nutrition on acid-base balance - metabolic aspects. Eur J Nutr 40, 214-220).

Recent searches indicate acid-base disorders and damaged renal functionality in subjects suffering from PKU who consume synthetic amino acid mixtures as a main protein source (Stroup, B.M., Sawin, E.A., Murali, S.G., Binkley, N., Hansen, K.E., Ney, D.M (2017). Amino Acid Medical Foods Provide a High Dietary Acid Load and Increase Urinary Excretion of Renal Net Acid Calcium, and Magnesium Compared with Glycomacropeptide Medical Foods in Phenylketonuria. J Nutr. Metab). Chronically compensating a high acid load resulting from the diet also increases calcium reabsorption from the urinary excretion and from bones, constituting a risk factor for the reduction of the bone mineral density.

Some studies indicate a reduction in bone mineralisation in 40-50% of adult patients and in about 33% of paediatric patients, with a bone mass reduced by at least 2 standard deviations if compared to the age expected range (Choukair, D., Kneppo, C., Feneberg, R. et al. (2017). Analysis of the functional muscle-bone unit of the forearm in patients with phenylketonuria by peripheral quantitative computed tomography. J Inherit Metab Dis, 40, 219-226).

Bone fragility therefore represents a possible complication in patients suffering from phenylketonuria and becomes particularly important considering the increase in the life expectancy of PKU patients treated with diet therapy from their birth thanks to the rise of neonatal screening.

There presently exists a need to regulate the dietary regime of subjects suffering from phenylketonuria such to maintain the acid-base balance within the physiological limits. At present, together with the observance of the dietary food regime, the diet therapy in PKU patients includes using protein substitutes which can be subdivided in three categories:

- Phenylalanine-free (Phe) synthetic amino acid mixtures;
- Phe-free synthetic amino acid mixtures, added with other nutrients such as vitamins, minerals and polyunsaturated fatty acids;
- Mixtures with Glycomacropeptide (GMP), a protein substitute derived from bovine milk serum, with a naturally reduced Phe content.

The use of common synthetic-based amino acid mixture formulations as a main protein source leads to the onset of acid-base balance disorders, due to an increase in the acidity load biasing the acid-base balance towards acidosis, which is related to the onset of the previously reported renal damages and bone fragility.

For this reason, there presently exists a need for having available phenylalanine-free synthetic amino acid mixture compositions whose formulation does not alter the physiological acid-base balance in subjects suffering from phenylketonuria.

A general object of the present invention consists in providing a nutritional or pharmaceutical composition for the treatment of metabolic diseases wherein an unbalance of the organism acid-base balance occurs.

Another object consists in providing a nutritional composition for the treatment of phenylketonuria whose formulation substantially prevents or reduces the onset of disorders of the human organism acid-base balance determined by a pH reduction of blood plasma and of other body liquids.

Another object consists in providing a nutritional composition for supplementing the dietary regime of subjects suffering from phenylketonuria in order to sensibly reduce or minimise the disorders of the acid-base balance resulting in a damage of renal functionality in subjects suffering from phenylketonuria consuming synthetic amino acid mixtures as a main protein source.

Still another object consists in providing a nutritional composition for supplementing the dietary regime of subjects suffering from phenylketonuria in order to maintain the acid-base balance and avoid or limit the reduction of the bone mineral density and the related increase of bone fragility.

SUMMARY OF THE INVENTION

The inventors, analysing studies on the population suffering from metabolic disorders which affect the organism acid-base balance, especially phenylketonuria, have observed that the acid-base balance alteration can be mainly related to an unbalance towards the use of synthetic amino acids recommended for these diseases.

In particular, the present invention originates from observing that the dietary regimes prescribed to the patent population suffering from phenylketonuria alter the acid-base balance and cause in many cases acidosis.

The inventors have thus found that it is feasible to maintain the acid-base balance in the patent population suffering from phenylketonuria, by formulating and administering synthetic amino acid-based compositions based on a defined ratio between sulfur amino acids (methionine and cysteine) and some specific minerals (sodium, potassium, magnesium, calcium, chlorine, phosphorus), in such amounts as to determine negative values of the PRAL (Potential Renal Acid Load) index, with values lower than -10 and preferably with values -35 < PRAL < -20 (values expressed as mEq/day on 100 g / PE, where PE means Equivalent Protein).

Advantageously, by fulfilling these PRAL values, the acid-base balance is kept within physiological values in the population suffering from phenylketonuria and renal damages and bone fragility reduce considerably.

According to some aspects of the present invention, the inventors have found that in the formulation of an amino acid and mineral-based composition intended for the dietary treatment of subjects suffering from phenylketonuria, the presence of selected sulfur amino acids as methionine and cysteine (and/or cystine), in specific ratios with selected minerals, determines negative PRAL values which keep the acid-base balance within physiological values, thus reducing occurrence of renal damages and bone fragility typical of this disease.

According to a first aspect, a composition is provided for use in the treatment of phenylketonuria, as defined in claim 1.

Advantageously, the composition for use in the treatment of phenylketonuria, in addition to the two sulfur amino acids, comprises the minerals sodium, potassium, magnesium, calcium whose presence contributes to determine a negative PRAL value and the additional minerals, phosphorus and chlorine.

Advantageously, the presence of methionine and cysteine in the composition in a methionine: cysteine ratio from 2.2 to 2.7 : 1 along with the presence of said minerals, advantageously within defined ranges, determines a negative PRAL value lower than - 10, preferably in the range from -35 to -20.

The composition for use of the invention may be a pharmaceutical composition, a food supplement or a dietary product which can be introduced in the dietary regime of a subject suffering from a metabolic disease in particular phenylketonuria.

Preferably the composition for use of the invention contains further amino acids and minerals suitable for preventing and/or treating phenylketonuria and at least a pharmaceutically acceptable or edible carrier.

The composition comprises a combination of methionine and cysteine in a methionine : cysteine ratio from 2.2 to 2.7 : 1 for use in the treatment of phenylketonuria.

According to some aspects, the inventors found that a composition containing a combination of methionine and cysteine in the herein described ratios and the minerals, keeps the acid-base balance of a human organism of a person suffering from phenylketonuria within the physiological parameters reducing the risk of side effects related to the intake of synthetic amino acid mixtures in the dietary regime.

The intake of the herein described composition in an amount which determines negative PRAL values, preferably lower than -10, in particular in the range from -35 to -20, significantly reduces the risk of developing renal damages and bone fragility in subjects suffering from phenylketonuria.

DETAILED DESCRIPTION OF THE INVENTION

According to certain aspects of the present invention, the inventors found out that the combination of two specific amino acids, such as methionine and cysteine, in a selected amount ratio with the herein disclosed minerals keep negative PRAL values. According to a first aspect, the present invention provides a composition, comprising sulfur amino acids and minerals, wherein said sulfur amino acids comprise a combination of methionine and cysteine in a methionine/cysteine ratio of 2.2-2.7: 1 preferably methionine : cysteine 2.4-2.6 : 1, more preferably methionine : cysteine 2.5 : 1, and said minerals comprise sodium, potassium, magnesium, calcium, chlorine, phosphorus, for use in the treatment of phenylketonuria.

Advantageously the combination of methionine and cysteine in the selected amount ratios, along with said minerals defined in specific ranges, contributes to determine a negative PRAL value which improves biochemical parameters and maintains the kidney and bones function of the organism of a subject suffering from phenylketonuria. Advantageously the composition of the invention is based on proteins and contains amino acids or peptides or further proteins with respect to methionine and cysteine, in particular one or more amino acids.

For exemplary purposes the composition of the invention may comprise other essential and non-essential amino acids.

Within the scope of the present invention the term PRAL refers to a parameter used to assess the metabolic impact of a food product, typically in terms of consequent alterations of the acid-base balance.

Typically, PRAL is calculated as (Remer T., Manz F. (1995). Potential Renal Acid Load of foods and its influence on urine pH. Journal of the American Dietetic Association. 95 (7), 791-797):

$$\text{PRAL (mEq/day)} = (0.49 \times \text{proteins g/d}) + 0.037 \times \text{phosphorus mg/d}) - (0.021 \times \text{potassium mg/d}) - (0.026 \times \text{magnesium mg/d}) - (0.013 \times \text{calcium mg/d}).$$

The PRAL model or parameter plays a key role because it foresees, in relation to the value obtained, positive or negative, the possible resulting alterations of the acid-base balance and of the plasmatic pH.

The PRAL model has been validated in dietary experiments and has shown to be strictly related to analysis of the values of excretion of renal net acid (NAE) (Alexy, U., Remer, T., Manz, F., Neu, C., Schoenau, E. (2005). Long-term protein intake and dietary potential renal acid load are associated with bone modelling and remodelling at the proximal radius in healthy children, The American Journal of Clinical Nutrition, 82 (5), 1107 - 1114).

To calculate the PRAL relative to amino acid mixtures administered to PKU patients, an alternative formula is used which takes into consideration sulphur amino acids methionine and cysteine, in addition to sodium, potassium, magnesium, calcium, chlorine, phosphorus (Stroup et al., 2017):

$$PRAL\ mEq/day = (2 \times (0.00503 \times mg\ Met/d)) + (2 \times (0.0062 \times mg\ Cys/d)) + (0.037 \times mg\ Phosphorus/d) + (0.0268 \times mg\ Chloride/d) - (0.021 \times mg\ Potassium/d) - (0.026 \times mg\ Magnesium/d) - (0.013 \times mg\ Calcium/d) - (0.0413 \times mg\ Sodium/d).$$

In case cystine is used, suitable conversion factors must be considered.

The composition of the invention contains specific minerals or macroelements which contribute to determine the negative PRAL and thus increase pH in blood (alkalosis) that are advantageously selected from sodium, potassium, magnesium and calcium and mixtures thereof.

Advantageously the composition for use as defined herein, contains the further minerals, phosphorus and chlorine.

In the composition for use of the invention sodium is preferably present in a concentration ranging from 0.6 to 1.0 % (w/w).

In the composition for use of the invention potassium is preferably present in a concentration ranging from 0.8 to 1.2 % (w/w).

In the composition for use of the invention magnesium is preferably present in a concentration ranging from 0.2 to 0.4 % (w/w).

In the composition for use of the invention calcium is preferably present in a concentration ranging from 0.9 to 1.3 % (w/w).

In the composition for use of the invention phosphorus is preferably present in a concentration ranging from 0.6 to 0.95 % (w/w).

In the composition for use of the invention chlorine is preferably present in a concentration ranging from 0.35 to 0.55 % (w/w).

Unless otherwise defined, all the technical and scientific terms used herein have the meaning as usually understood by a person skilled in the art of the present invention. The term PRAL represents the acronym for Potential Renal Acid Load.

As used herein, the term "combination" indicates that one or more of the biologically active components, such as sulfur amino acids or minerals, are added or mixed with one or other ingredients. The term "combination" does not mean that amino acids are associated between each other forming chemical bonds or other types of bonds.

The term "macroelement" indicates physiologically acceptable minerals which, typically, are present in the human organism in such amounts as to contribute to a biologic activity or to act in a cell action mechanism.

The term "metabolic acidosis", is meant to comprise forms of acidosis due to a primary reduction in bicarbonate body concentration. It is typically a disorder of the acid-base balance which tends to reduce the pH in blood plasma and other body liquids.

In particular, as used herein, the term "edible" indicates eatable substances whose use in the formulation of a nutritional or food composition to be administered orally has been approved by health authorities.

The terms "edible, eatable, physiologically acceptable" refer to substances which are typically used in the formulation of nutritional, food or pharmaceutical products. A "physiologically acceptable" carrier may be a pharmaceutically acceptable carrier. The term "carrier" as used herein indicates a means, an excipient, an extender with which the combination of active components of the formulation is administered.

In some embodiments the composition comprises an edible and/or physiologically acceptable carrier.

The herein disclosed composition is formulated with carriers suitable for oral administration.

The biologically active substances such as amino acids and minerals, contained in the composition can be combined or mixed as active ingredients in a thorough mixture with a carrier and/or eatable excipient according to the pharmaceutical and food industry or traditional nutritional techniques.

Any carrier and/or excipient suitable for the desired preparation form to be administered to humans is contemplated for use with the compounds described in the present invention.

In some embodiments, the composition of the invention comprises biologically active substances or added active ingredients.

For example, the composition for the use of the invention may include one or more vitamins, such as vitamins of group the B, vitamin A, vitamin C vitamin D, and so on. According to some embodiments, the composition for use of the invention comprises the micronutrients and/or minerals as corresponding anions or cations or in salt form. In some embodiments additional minerals can be present selected from iron, zinc, manganese, copper, selenium, etcetera and mixtures thereof.

In some embodiments the composition for use of the invention further contains essential and non-essential amino acids and mixtures thereof.

The compositions for use of the invention are suitable for food, nutritional, dietary or pharmaceutical use in mammals, in particular in humans.

The composition for use of the invention may have a wide variety of physical and/or pharmaceutical forms of preparation, according to the desired route of administration. The composition for use of the invention may be in solid, liquid or semiliquid form, preferably solid.

When the composition for use of the invention is in solid form it can be in the form of tablet, capsule, powder, granules, pill, lozenge and powders and is preferably in granule or powder form.

Preparation in solid form may comprise one or more excipients/carriers such as amides, sugars, microcrystalline cellulose and optionally diluents, granulating agents, lubricants, ligands, disintegrating agents.

Typically, the solid-form composition may contain a ligand such as tragacanth, gum, corn starch or gelatine; excipients such as dicalcium phosphate, a disintegrating agent such as corn starch, potato starch, alginic acid; a lubricant such as magnesium stearate; a sweetener such as sucrose, lactose or saccharin.

According to some embodiments the composition for use of the invention contains a cellulose-based excipient which comprises i) cellulose organic esters, for instance selected from cellulose acetate, cellulose propionate, cellulose triacetate, cellulose acetate propionate, cellulose acetate butyrate, ii) cellulose inorganic esters, for instance selected from, nitrocellulose, cellulose sulfate, iii) cellulose ethers selected from a) cellulose alkyl ethers selected for instance from, methyl cellulose, ethyl cellulose; b) cellulose hydroxyalkyl ethers selected for instance from hydroxyethyl cellulose, hydroxypropyl cellulose, hydroxypropyl methylcellulose, ethyl hydroxyethyl cellulose; c) carboxyalkyl cellulose, such as, carboxymethylcellulose, salts thereof and mixtures thereof. In certain embodiments the excipients based on cellulose are crosslinked with physiologically acceptable crosslinking agents.

In some embodiments, the composition for use of the invention further comprises one or more additional components such as additives, fillers, stabilizers, emulsifiers, texturizers, film-forming agents, plasticizers, wetting agents and thickeners.

In the composition for uses herein disclosed, there may also be flavouring agents, preservatives, colouring agents.

In certain embodiments, the composition for use of the invention comprises as an excipient a hydrogenated fatty acid, preferably having a chain with from 3 to 20 carbon atoms, from 14 to 18 carbon atoms. A typical example of a hydrogenated fatty acid is the hydrogenated palm oil.

The composition for use of the invention may be in liquid form.

When the composition is in liquid form, it can be in form of suspension, emulsion, solution, oral spray, mouthwash. In these cases, the carrier is liquid and may be selected from water, glycols, oils, alcohols and mixtures thereof.

Typically, when the formulation is in liquid form it may contain excipients such as sucrose as a sweetener agent, methyl- and propyl-parabens as preservatives, a colouring agent and a flavour such as cherry or orange.

The combined compositions can be suitably formed in a single pharmaceutical form or unit of dosage with dosages of the ingredients as defined herein or in the appended claims 3, 4, and prepared using any conventional methods of the pharmaceutical or food field.

In some embodiments, in the compositions of the present invention, the active ingredients are usually formulated in a dosage unit. The dosage unit can contain from 0.1 to 1,000 mg of each active ingredient per dosage unit for daily administration.

In some embodiments the dose is in the range from 0.001 % by weight to about 60% by weight of the formulation.

According to some embodiments, the composition for use of the invention is a nutraceutical, a food supplement, a nutritional product, a dietary product, a special medical purpose food supplement or food product.

Typically, the composition for use according to the present invention is administered orally.

Preferably, for the oral administration the composition for use of the invention is in powder or granulate form.

The amount administered, and the administration rate and time trend will depend on the nature and severity of the phenylketonuria to be treated. The treatment prescription, such as, the decisions about the dosage etc., fall within the responsibility and the choice of physicians and dieticians, and it typically takes in consideration the condition to be treated, the condition of the single subject, place of delivery, administration method and other factors known to professionals. The precise dose will depend on a number of factors, including the composition form to be administered.

In some embodiments the composition for the use as herein disclosed is administered once or more times a day to a subject requiring the treatment.

The following examples are provided for illustrative purposes of the invention.

EXAMPLE 1

Composition for use in the treatment of phenylketonuria or to maintain the acid-base balance in a subject, having the following formulation:

| Components | Quantity |
| --- | --- |
| Equivalent Proteins, g | 100 g |
| Methionine, mg | 2500 mg |
| Cysteine, mg | 1000 mg |
| Phosphorus, mg | 1417 mg |
| Chlorine, mg | 833 mg |
| Potassium, mg | 1833 mg |
| Magnesium, mg | 583 mg |
| Calcium, mg | 1997 mg |
| Sodium, mg | 1332 mg |
| Pral, mEq | -22.3 |

EXAMPLE 2

Comparative Example

The Applicant has analysed the formulation and calculated the PRAL of protein-based formulations which do not match the quality and quantitative characteristics of the compositions of the invention.

Hereinafter formulations of the compositions for the use of the invention and comparative nutritional products/supplements and the related PRAL values are compared.

Analyses have shown that the compositions having formulations different from the invention have a positive PRAL, whereas the compositions for use of invention such as the one indicated in Example 1 have a negative PRAL.

It is clear by the comparison that a negative PRAL is obtained as requested only with the composition for use of the invention.

| | COMPOSITION OF THE INVENTION | | COMPARATIVE PRODUCT 1 | | COMPARATIVE PRODUCT 2 | |
| --- | --- | --- | --- | --- | --- | --- |
| Equivalent Proteins, g | 60 | 100 | 60 | 100 | 28 | 100 |
| Methionine, mg | 1500 | 2500 | 1240 | 2067 | 730 | 2607 |
| Cysteine, mg | 600 | 1000 | 1670 | 2783 | 1900 | 6786 |
| Phosphorus, mg | 850 | 1417 | 1068 | 1780 | 708 | 2529 |
| Chlorine, mg | 500 | 833 | 728 | 1213 | 448 | 1600 |
| Potassium, mg | 1100 | 1833 | 940 | 1567 | 490 | 1750 |
| Magnesium, mg | 350 | 583 | 376 | 627 | 123 | 439 |
| Calcium, mg | 1198 | 1997 | 1196 | 1993 | 1078 | 3850 |
| Sodium, mg | 799 | 1332 | 508 | 847 | 308 | 1100 |
| Pral, mEq | -22.3 | | 43.6 | | 103.1 | |

|  | PRODUCT 3 | | PRODUCT 4 | | PRODUCT 5 | |
|---|---|---|---|---|---|---|
| Equivalent Proteins, g | 72 | 100 | 60 | 100 | 40 | 100 |
| Methionine, mg | 1400 | 1944 | 1500 | 2500 | 830 | 2075 |
| Cysteine, mg | 2200 | 3056 | 1500 | 2500 | 1110 | 2775 |
| Phosphorus, mg | 992 | 1378 | 1380 | 2300 | 650 | 1625 |
| Chlorine, mg | 5 | 7 | 902 | 1503 | 450 | 1125 |
| Potassium, mg | 25 | 35 | 1200 | 2000 | 450 | 1125 |
| Magnesium, mg | 384 | 533 | 315 | 525 | 120 | 300 |
| Calcium, mg | 1280 | 1778 | 2299 | 3832 | 1100 | 2750 |
| Sodium, mg | 20 | 28 | 541 | 902 | 200 | 500 |
| Pral, mEq | 69.8 | | 38.8 | | 57.7 | |

|  | PRODUCT 6 | | PRODUCT 7 | | PRODUCT 8 | |
|---|---|---|---|---|---|---|
| Equivalent Proteins, g | 70 | 100 | 69 | 100 | 63.2 | 100 |
| Methionine, mg | 1500 | 2143 | 1400 | 2029 | 1100 | 1741 |
| Cysteine, mg | 2000 | 2857 | 2000 | 2899 | 1500 | 2373 |
| Phosphorus, mg | 733 | 1047 | 824 | 1194 | 950 | 1503 |
| Chlorine, mg | 0 | 0 | 0 | 0 | 690 | 1092 |
| Potassium, mg | 0 | 0 | 7 | 10 | 1111 | 1758 |
| Magnesium, mg | 312 | 446 | 309 | 448 | 350 | 554 |
| Calcium, mg | 1090 | 1557 | 1236 | 1791 | 1200 | 1899 |
| Sodium, mg | 0 | 0 | 275 | 399 | 25.1 | 40 |
| Pral, mEq | 63.9 | | 48.9 | | 54.2 | |

**Claims**

1. A composition, comprising sulfur amino acids and minerals, wherein said sulfur amino acids comprise a combination of methionine and cysteine in a ratio of 2.2-2.7: 1, preferably 2.4-2.6: 1, preferably 2.5: 1 and the minerals comprise sodium, potassium, magnesium, calcium, chlorine, phosphorus, for use in the treatment of phenylketonuria.

2. The composition for use according to claim 1, **characterized in that** it has a negative PRAL value, preferably less than -10, preferably in a range comprised from -35 to -20, calculated in mEq/day on 100 g/equivalent proteins.

3. The composition for use according to claims 1 or 2, in a dosage unit wherein the methionine is contained in an amount in the range from 2330 mg to 2830 mg with respect to an amount of 100 g of equivalent proteins.

4. The composition for use according to claim 3, in a dosage unit wherein the cysteine is contained in an amount in the range from 930 mg to 1130 mg with respect to an amount of 100 g of equivalent proteins.

5. The composition for use according to any one of claims 1-4 further comprising amino acids or proteins.

6. The composition for use according to claim 5 wherein the additional amino acids are selected from essential and non-essential amino acids and mixtures thereof.

7. The composition for use according to any one of claims 1-6, wherein it is provided in a form for oral administration.

8. The composition for use according to claim 7, wherein it is provided in solid form of powder, tablet, capsule, granules, pill or in liquid form of solution, suspension, or emulsion.

9. The composition for use according to claim 8 wherein it is provided in the form of powder or granules.

Europäisches
Patentamt

European
Patent Office

Office européen
des brevets

**EUROPEAN SEARCH REPORT**

Application Number

EP 21 16 9147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/317597 A1 (NEY DENISE M [US] ET AL) 16 December 2010 (2010-12-16)<br>* table 1 * | 1-9 | INV.<br>A61K31/198<br>A61K33/20<br>A61K33/42<br>A61P43/00 |
| A | US 2020/069624 A1 (REINER ALBERTO [IT] ET AL) 5 March 2020 (2020-03-05)<br>* page 15; table 14 * | 1-9 | |
| A | WO 2016/003263 A1 (NUTRICIA NV [NL])<br>7 January 2016 (2016-01-07)<br>* abstract * | 1-9 | |
| A | "Special diets for infants with inborn errors of amino acid metabolism",<br>PEDIATRICS, AMERICAN ACADEMY OF PEDIATRICS, US,<br>vol. 57, no. 5, 30 April 1976 (1976-04-30)<br>, pages 783-792, XP009524841,<br>ISSN: 0031-4005 | 1-9 | |
| E | RU 2 752 901 C2 (FEDERALNOE GOSUDARSTVENNOE BYUDZHETNOE OBRAZOVATELNOE UCHREZHDENIE VYS)<br>11 August 2021 (2021-08-11)<br>* abstract * | 1,2,5-7 | **TECHNICAL FIELDS SEARCHED (IPC)**<br><br>A61K<br>A61P |

-/--

The present search report has been drawn up for all claims

| Place of search | Date of completion of the search | Examiner |
|---|---|---|
| The Hague | 6 September 2021 | Bonzano, Camilla |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

## EUROPEAN SEARCH REPORT

Application Number

EP 21 16 9147

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | STROUP BRIDGET M. ET AL: "Amino Acid Medical Foods Provide a High Dietary Acid Load and Increase Urinary Excretion of Renal Net Acid, Calcium, and Magnesium Compared with Glycomacropeptide Medical Foods in Phenylketonuria", JOURNAL OF NUTRITION AND METABOLISM, vol. 2017, 1 January 2017 (2017-01-01), pages 1-12, XP055837938, US ISSN: 2090-0724, DOI: 10.1155/2017/1909101 Retrieved from the Internet: URL:https://www.ncbi.nlm.nih.gov/pmc/artic les/PMC5436062/pdf/JNME2017-1909101.pdf> ----- | | |

TECHNICAL FIELDS
SEARCHED      (IPC)

The present search report has been drawn up for all claims

| Place of search | Date of completion of the search | Examiner |
|---|---|---|
| The Hague | 6 September 2021 | Bonzano, Camilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                  

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 16 9147

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2021

| Patent document cited in search report | | Publication date | Patent family member(s) | | | Publication date |
|---|---|---|---|---|---|---|
| US 2010317597 | A1 | 16-12-2010 | AU | 2010259975 | A1 | 22-12-2011 |
| | | | BR | PI1013089 | A2 | 05-04-2016 |
| | | | CA | 2764571 | A1 | 16-12-2010 |
| | | | CL | 2011003120 | A1 | 25-05-2012 |
| | | | CN | 102481338 | A | 30-05-2012 |
| | | | CN | 106983854 | A | 28-07-2017 |
| | | | DK | 2440232 | T3 | 30-04-2018 |
| | | | EP | 2440232 | A1 | 18-04-2012 |
| | | | ES | 2669094 | T3 | 24-05-2018 |
| | | | JP | 2012530067 | A | 29-11-2012 |
| | | | NZ | 596902 | A | 27-09-2013 |
| | | | PT | 2440232 | T | 23-03-2018 |
| | | | US | 2010317597 | A1 | 16-12-2010 |
| | | | US | 2013196024 | A1 | 01-08-2013 |
| | | | US | 2014248414 | A1 | 04-09-2014 |
| | | | WO | 2010144821 | A1 | 16-12-2010 |
| US 2020069624 | A1 | 05-03-2020 | AU | 2019326896 | A1 | 25-03-2021 |
| | | | BR | 112021000683 | A2 | 13-04-2021 |
| | | | CA | 3105341 | A1 | 05-03-2020 |
| | | | CN | 112805061 | A | 14-05-2021 |
| | | | CO | 2021002386 | A2 | 08-03-2021 |
| | | | EP | 3843839 | A1 | 07-07-2021 |
| | | | TW | 202025997 | A | 16-07-2020 |
| | | | US | 2020069624 | A1 | 05-03-2020 |
| | | | WO | 2020044163 | A1 | 05-03-2020 |
| WO 2016003263 | A1 | 07-01-2016 | AU | 2015284857 | A1 | 19-01-2017 |
| | | | CA | 2953768 | A1 | 07-01-2016 |
| | | | CN | 106686990 | A | 17-05-2017 |
| | | | DK | 3164124 | T3 | 04-02-2019 |
| | | | EP | 3164124 | A1 | 10-05-2017 |
| | | | US | 2017143025 | A1 | 25-05-2017 |
| | | | WO | 2016003263 | A1 | 07-01-2016 |
| | | | WO | 2016003273 | A1 | 07-01-2016 |
| RU 2752901 | C2 | 11-08-2021 | ---------------------------------------- | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VAN WEGBERG, A.M.J. ; MACDONALD, A. ; AHRING, K. et al.** The complete European guidelines on phenylketonuria: diagnosis and treatment. *Orphanet J Rare Dis,* 2017, vol. 12, 162
- **REMER, T.** Influence of nutrition on acid-base balance - metabolic aspects. *Eur J Nutr,* 2001, vol. 40, 214-220
- **STROUP, B.M. ; SAWIN, E.A. ; MURALI, S.G. ; BINKLEY, N. ; HANSEN, K.E. ; NEY, D.M.** Amino Acid Medical Foods Provide a High Dietary Acid Load and Increase Urinary Excretion of Renal Net Acid Calcium, and Magnesium Compared with Glycomacropeptide Medical Foods in Phenylketonuria. *J Nutr. Metab,* 2017
- **CHOUKAIR, D. ; KNEPPO, C. ; FENEBERG, R. et al.** Analysis of the functional muscle-bone unit of the forearm in patients with phenylketonuria by peripheral quantitative computed tomography. *J Inherit Metab Dis,* 2017, vol. 40, 219-226
- **REMER T. ; MANZ F.** Potential Renal Acid Load of foods and its influence on urine pH. *Journal of the American Dietetic Association,* 1995, vol. 95 (7), 791-797
- **ALEXY, U. ; REMER, T. ; MANZ, F. ; NEU, C. ; SCHOENAU, E.** Long-term protein intake and dietary potential renal acid load are associated with bone modelling and remodelling at the proximal radius in healthy children. *The American Journal of Clinical Nutrition,* 2005, vol. 82 (5), 1107-1114